# EUROPEAN PATENT SPECIFICATION

(11) **EP 4 143 161 B1**
(45) Date of publication and mention of the grant of the patent: **01.11.2023**
(21) Application number: 21717902.7
(22) Date of filing: 14.04.2021
(51) Int. Cl.: C07C 319/22, C07C 319/28, C07C 323/59

(54) **PROCESS OF MAKING N,N-DIACETYL-L-CYSTINE DISODIUM SALT FROM CYSTINE AND ACETYL CHLORIDE IN METHANOL IN THE PRESENCE OF SODIUM HYDROXIDE**
VERFAHREN ZUR HERSTELLUNG VON N,N-DIACETYL-L-CYSTIN-DINATRIUMSALZ AUSGEHEND VON CYSTIN UND ACETYLCHLORIDE IN METHANOL IN GEGENWART VON NATRIUMHYDROXID
PROCÉDÉ DE FABRICATION DU SEL DISODIQUE DE N,N-DIACÉTYL-L-CYSTINE À PARTIR DE CYSTINE ET CHLORURE D'ACÉTYL EN MÉTHANOL EN PRÉSENCE DE HYDROXYDE DE SODIUM

(30) Priority: 28.04.2020 EP 20171852
(43) Date of publication of application: 08.03.2023
(73) Proprietor: Unilever IP Holdings B.V., 3013 AL Rotterdam (NL); Unilever Global IP Limited, Wirral, Merseyside CH62 4ZD (GB)
(72) Inventor: ROSA, Jose, Guillermo, Trumbull, CT 06611 (US); HARICHIAN, Bijan, Trumbull, CT 06611 (US)
(74) Representative: James, Helen Sarah
(86) International application number: PCT/EP2021/059652
(87) International publication number: WO 2021/219377

(56) References cited:
- CN-A- 108 558 721

## Description

### Field of the invention

The present invention is directed to an efficient method of making N,N'-diacetyl-L-Cystine disodium salt ("NDAC disodium salt") directly from cystine using an acetylating agent.

### Background of the invention

Healthy look is a universal consumer need leading to their personal care. Healthy looking skin can be described in terms of attributes of appearance (glow, radiance, evenness of hue, pigmentation spots), texture (smoothness, silkiness, lack of bumps and pores), and age (fine lines, wrinkles, elasticity, and sagging/laxity). While different classes of compounds claim cosmetic benefits on skin appearance, cysteine and cystine derivatives have not received much attention.

A number of cysteine/cystine derivatives, including β-substituted cysteine/cystines, cystine diamides, cystine dialkyl esters and N-alkanoylcysteines have potential therapeutic benefits, for example in kidney stone prevention (See , Zhu, et al., "Rational Design of Novel Crystal Growth Inhibitors for Treatment of Cystinuria Kidney Stones," 2013 ProQuest Dissertations and Theses; CrystEngComm, 2016, 18, 8587). However, even the simplest derivative of cystine, N,N'-diacetyl-L-cystine ("NDAC"), lacks industrial application, especially in the beauty and personal care field.

As a result of few known uses, NDAC and/or NDAC salts are practically not available on a commercial scale. It has now been discovered that NDAC salt breaks down into cystine, which is reduced to cysteine in cells of an organism, i.e., intracellular formation of cysteine. Cysteine and its dimer Cystine are glutathione precursors.

Glutathione (GSH) is a tripeptide that consists of glutamate, cysteine, and glycine. GSH is present in all mammalian tissues. It is the main antioxidant in the living body, whereby it protects cells from oxidation by quenching reactive oxygen species. GSH is believed to play a significant role in protecting cells against the cytotoxic effects of ionizing radiation, heat, certain chemicals, and solar UV radiation. While true in all areas of the body, this is particularly important in the skin, which is exposed to damaging effects of radiation, particularly UV radiation, and environmental pollutants. Decrease in the intracellular concentration of glutathione in skin is associated with cell damage, inflammation, skin darkening, discoloration, spots or freckles caused by exposure to ultraviolet radiation, and overall physiological aging.

Compositions for potentiating intracellular glutathione production have been described. See e.g. Chiba et al. US Patent 7,740,831, Crum et al (USRE37934, USRE42645, WO2016/033183, and US20050271726); Mammone US Patent 6,149,925, and Perricone US 20060063718. Topical compositions and enhancing generation of glutathione in skin from its constituent amino acids (glutamate, cysteine, and/or glycine, i.e., glutathione precursors) for cellular uptake and synthesis of the GSH tripeptide was addressed in, e.g., Applicant's U.S. Published Patent Application Nos.: US20/9034, US20/16059, and US19/328631.

Applicant has now discovered that the performance of NDAC is comparable to that of cystine for potentiating glutathione synthesis within skin cells, but NDAC is more soluble and can have better delivery to skin, thereby resulting in better performance. Proteases in the body can cleave (or hydrolyze) the N-acetyl bonds of NDAC to ultimately generate cystine, thus, NDAC can also serve as a controlled release alternate source of cystine. Accordingly, there is a need for the NDAC salt material.

Examples of other cystine derivatives which are less advantageous than NDAC are N-Acetyl Cysteine ("NACys") which contains a thiol (SH) group and L-Cystine diethyl ester ("DEC") which does not contain an SH-group. NACys is a thiol drug used commonly as an expectorant (Cryst.Eng.Comm., 2016, 18, 8587, referred to as "NACe" therein). NACys provides an upleasant sulfurous odor, that is unacceptable in cosmetic products. Furthermore, the sulfurous odor (monitored as hydrogen sulfide or H₂S) is consistent with NACys decomposition which is also unacceptable in marketed cosmetic products. Similarly, DEC also generates a strong undesirable sulfurous odor and is not stable in formulated cosmetic products.

NDAC is an amide, i.e., an N,N'-diacetyl derivative of cystine. NDAC, for purposes of the present invention, has the following chemical structure:

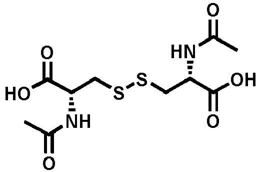

The molecular weight of NDAC = 324.4.

NDAC stereoisomers (referring to the stereoisomerism of the alpha-Carbon atom located between the Nitrogen atom and the Carbonyl group of the carboxylic acid moiety) of the present invention include R,R (L-cystine), R,S, S,R and S,S (D-cystine). Preferably, L stereoisomers are employed, and this is the most abundant and natural isomeric form found in nature.

NDAC is not readily commercially available but may be sourced on lab scale. NDAC may be synthesized directly from NACys as described in Vandana Rathore et al, Organic Letters, 20(19), 6274-6278; 2018 and Scott J. Pye et al, Green Chemistry, 20(1), 118-124; 2018:

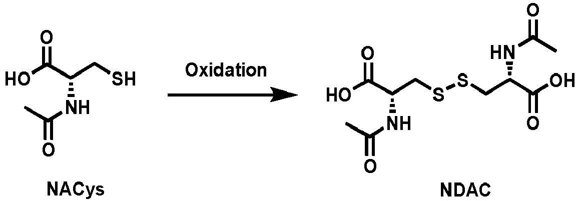

Such chemical processes involving oxidative dimerization of NACys, or other SH-containing raw materials, to generate NDAC suffer from significant disadvantages. First, any unreacted NACys is difficult to remove from the desired NDAC product and requires specialized purification methods such as chromatography using polar solvent systems. Second, such purification methods are impractical at very large scales (e.g. kg, tons). Third, even very small amounts of NACys impurities in the final NDAC product will render it unusable for cosmetic products as SH-containing impurities such as NACys will generate undesirable sulfurous odor when formulated. Fourth, during oxidation of NACys into NDAC can lead to premature oxidation of NACys and overoxidation of NDAC into undesirable and difficult to remove sulfenic/sulfenic acids, sulfone and/or sulfoxide impurities. Therefore, a chemical process that avoids the use of thiol-containing raw materials and/or oxidizing conditions is highly desirable.

A sensible approach that avoids the use of thiol-containing raw materials with the potential to generate cystine derivatives like NDAC involves the use of cystine or cystine-derived raw materials. A practical process involving the direct conversion of cystine into NDAC has not been reported, mainly due to cystine's extremely low solubility in water and organic solvents (e.g. the solubility of cystine in water is 0.112 mg/ml at 25°C; cystine is more soluble in aqueous solutions with pH less than 2 or pH above 8; cystine is practically insoluble in organic solvents such as alcohols, ethers, esters, ketones, etc.).

CN 108 558 721 A (UNIV JILIN) discloses a process for preparing the N,N'-diacetyl-L-cystin disodium salt from L-cystine in an aqueous solution in the presence of sodium hydroxide and acetic anhydride.

More complex than NDAC, higher molecular weight N,N'-dialkanoyl cystines have been chemically prepared directly from cystine and higher molecular weight acid chlorides (see, e.g., Journal of Chromatography (1989) 483, 239-252; Journal of Materials Chemistry B (2015) 3, 7262-7270; Applied Materials and Interfaces (2016) 8, 21155-21162; Organic and Biomolecular Chemistry (2017) 15, 3840-3842; Journal of Dispersion Science and Technology (2014) 35, 1051-1059; Journal of Medicinal Chemistry (2008) 51, 684-688; Faming Zhuanli Shenqing, 102093468). Higher molecular weight anhydrides are also used as reagents (see for example Faming Zhuanli Shenqing Gongkai Shuomingshu, 1810769). Further, high molecular weight activated carboxylic acids can also be used as reagents (see for example Bioconjugate Chemistry (2015) 26, 145152; ACS Chemical Biology (2009) 4, 547-556; US 20120232120; Biomacromolecules (2015) 16, 2436-2443). While yields are acceptable, unfortunately, most of these methods use undesirable organic solvents at the commercial scale such as tetrahydrofuran, N,N-dimethylformamide, dichloromethane and/or mixed solvents systems (e.g. Schotten-Baumann conditions). These organic solvents are toxic and/or flammable, and, in any event, require removal at the end of the reaction. Furthermore, the existing processes frequently result in the formation of by-products which also require complicated and labor-intensive removal, such as by multi-step extraction methods or chromatography.

On the other hand, processes to chemically prepare N,N'-dialkanoyl cystine products from low molecular weight acid chlorides (e.g. acetyl chloride), anhydrides (e.g. acetic anhydride) or other activated carboxylic acids suffer from low yields and require complex methods of purification. This is due in part to the higher reactivity of low molecular weight acid chlorides and anhydrides (and higher potential for decomposition when exposed to basic aqueous media required to solubilize cystine to achieve a chemical reaction between them in solution).

Therefore, there is a commercial need for a chemical process to specifically prepare N,N'-diacetyl-L-Cystine ("NDAC") disodium salt (2) directly from cystine (1) using low molecular weight acetylating agents (e.g. acetyl chloride or acetic anhydride).

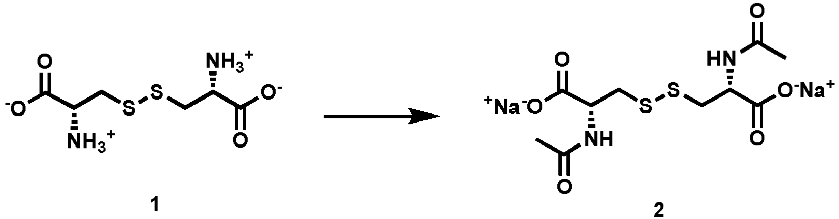

There is a need in the art for a practical chemical process to generate NDAC disodium salt directly from cystine using a low molecular weight acid chloride or anhydride, that is also "green" or without generating organic or otherwise harmful by-products, generates no waste (or a minimum amount of waste) and the reagents/solvents used in the process can be recovered and recycled.

### SUMMARY OF THE INVENTION

The present invention obviates the above-described need in the art by providing a chemical process to specifically prepare N,N'-diacetyl-L-Cystine ("NDAC") disodium salt (2) directly from cystine (1) using an acetylating agent (e.g. acetyl chloride or acetic anhydride).

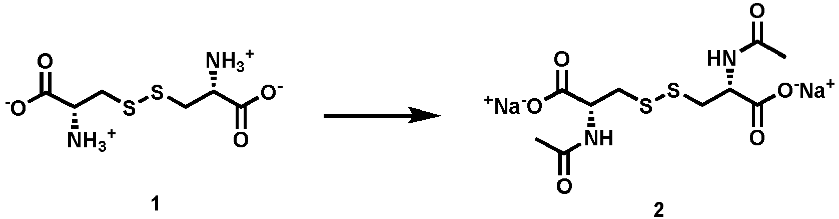

The chemical process of the present invention is quick and is performed in a single vessel in one step, when a batch process is used. With either batch or continuous process, the inventive reaction advantageously starts with cystine, and uses alcohol-based solvents which can be recycled. There are no undesirable cystine-derived by-products generated and any by-products generated consists of innocuous salts (e.g. NaCl) that can be conveniently filtered off and recycled or small amounts of acceptable by-products consisting of cystine disodium salt (from unreacted cystine), N-acetyl-L-cystine sodium salt (resulting from monoacetylation of cystine) and sodium acetate. The N,N'-diacetyl-L-Cystine ("NDAC") disodium salt (2) product is isolated upon solvent evaporation, with no purification needed.

The present invention includes a single-step process of making N-diacetyl-L-cystine disodium salt ("NDAC disodium salt") the process comprising:
adding L-cystine to a cold (~10 °C) solution of methanol (5ml) containing sodium hydroxide (333mg, 8.3mmol) and stirring for 5 min to form a solution;
cooling the solution to 5 °C and adding acetyl chloride (0.3ml, 4.2mmol) portionwise, while maintaining the temperature below 10 °C, thereby resulting in a white suspension;
stirring the resulting white suspension at 5 °C for 20 min and allowing said suspension to warm up to R.T. (~20 °C) over 10 min, thereby resulting in N,N'-diacetyl-L-cystine disodium salt product.

In one aspect, a process of making N,N'-diacetyl-L-cystine disodium salt comprises:
Mixing a hydroxyalkane (5 ml) with a sodium base (4 mmol equivalents per mmol of cystine) and cooling to form a cold solution at a temperature of 10°C;
Adding cystine (1 mmol equivalent) to said cold (~10 °C) solution and stirring for 5 min to form a cystine basic solution;
Cooling the said cystine basic solution to 5 °C and adding acetyl chloride (2.0 mmol equivalents per mmol of cystine) portionwise, while maintaining the temperature below 10 °C, thereby resulting in a white suspension;
Stirring said white suspension at 5 °C for 20 min and allowing said suspension to warm up to R.T. (~20 °C) over 10 min, until the reaction is completed, thereby resulting in N,N'-diacetyl-L-cystine disodium salt product dissolved in solution and sodium chloride by-product precipitated solid in said suspension.

In a larger scale alternative process of making N,N'-diacetyl-L-Cystine disodium salt, the process includes:
(i) Mixing **hydroxyalkane** (between 0.5-100L, preferably between 0.5-10L, more preferably between 1-3L, most preferably 1L per mol of cystine) with a sodium base (4.0 molar equivalents per mole of cystine) to form a cold solution at a temperature of from 5 to 10°C;
(ii) Adding **cystine** (1 molar equivalent) to said cold solution and stirring, for a sufficient time, to form a basic **cystine solution;**
(iii) Optionally, Cooling the **cystine solution** to 5°C;
(iv) Adding acetyl chloride (2 molar equivalents per mole of cystine) portionwise, while maintaining the temperature between 3 and 50 °C, preferably between 5 and 35 °C, more preferably below 10°C, most preferably 5°C, thereby resulting in a white suspension;
(v) **Stirring said white suspension** and allowing said suspension to warm up to a room temperature of 15°C to 50°C, preferably 20°C to 35°C, more preferably 20°C,
thereby resulting in N,N'-diacetyl-L-Cystine disodium salt product dissolved in solution and sodium chloride by-product precipitated solid in said suspension.

Further, the process may include separating the NDAC disodium salt (2) product from the reaction mixture. In particular, the process may include:
Filtering off said sodium chloride by-product precipitated solid, thereby separating said precipitated solid away from N,N'-diacetyl-L-Cystine disodium salt product dissolved in filtrate,
Washing said precipitated solids with anhydrous methanol and collecting said methanol filtrate,
Combining all filtrates,
Evaporating said combined filtrate under reduced pressure and temperature of 40°C, resulting in N,N'-diacetyl-L-cystine disodium salt (2) product as white solid at 90% yield.

The inventive process is fast, does not require labor-intensive isolation or purification of the product, NDAC, by removal of organic solvent or by-products, has improved yield and purity and by-products and solvent can be recycled and reused.

A general procedure for making N,N'-diacetyl-L-cystine salts is as follows.

L-Cystine (1 molar equivalent, 500mg, 2.1mmol) is added to a solution of a hydroxyalkane or a dihydroxyalkane or a trihydroxyalkane, preferably hydroxyalkane, at a concentration of between 0.5ml-100ml of hydroxyalkane (or dihydroxyalkane or trihydroxyalkane) per mmol of L-cystine, containing and alkali metal hydroxide such as for example sodium hydroxide (preferably an alkali metal hydroxide, most preferably sodium hydroxide) in 4-6 molar equivalents with respect to cystine, or alkaline earth hydroxides or oxides such as for example calcium hydroxide or calcium oxide with a pH ranging from 9-15 (preferably between 10-14, most preferably less than 14) at temperatures ranging between 5-50 °C (preferably between 5-35 °C, more preferably between 5-25 °C, most preferably less than about 10 °C) and stirred anywhere between 5 min to 2h, depending on the scale of the process, or the amount of reagents, and the corresponding mixing time needed to generate a homogeneous solution/mixture.

Suitable hydroxyalkanes include, for example methanol, ethanol or isopropanol (preferably methanol). Suitable dihydroxyalkane include, for example, propylene glycol. Suitable trihydroxyalkanes include, for example, glycerol.

The solution is cooled to between 5-25 °C and an acetylating agent such as for example acetyl chloride or acetic anhydride (preferably acetyl chloride, most preferably acetyl chloride) (2-4 molar equivalents with respect to cystine, preferably 2-3 molar equivalents, most preferably 2 molar equivalents) is added slowly or in portions, maintaining the temperature below 50 °C (preferably below 30 °C, more preferably below 20 °C, most preferably less than about 10 °C). In one embodiment, acetyl chloride (AcCI) is added at room temperature (R.T.) of about 15 °C to about 20°C.

The reaction mixture is stirred below 50 °C (preferably below 30 °C, more preferably below 20 °C,most preferably less than about 10 °C) until all the cystine is consumed (typically between 15min-24h) and the desired N,N'-diacetyl-L-cystine salt is generated.

The precipitated solids are filtered off and washed with a hydroxyalkane (preferably an alcohol, most preferably methanol) and the combined filtrates evaporated to yield N,N'-diacetyl-L-cystine salt as a solid.

A reaction scheme in accordance with the present invention is shown below:

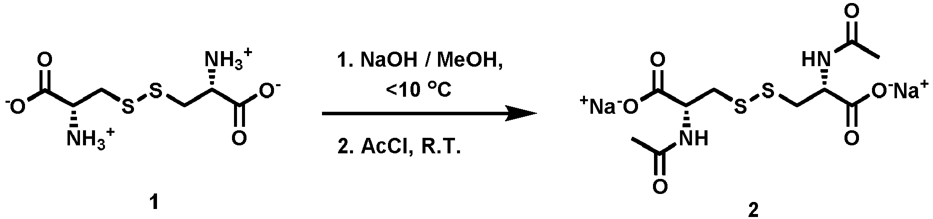

In one specific embodiment, N,N'-diacetyl-L-cystine disodium salt is prepared as follows. L-Cystine (1) (500mg, 2.1mmol) is added to a cold (~10 °C) solution of methanol (5ml) containing sodium hydroxide (333mg, 8.3mmol) and stirred for 5 min. The solution is cooled to 5 °C and acetyl chloride (0.3ml, 4.2mmol) added portionwise, maintaining the temperature below 10 °C. The resulting white suspension is stirred at 5 °C for 20 min and allowed to warm up to R.T. (room temperature, about 20°C) over 10 min. At this time, TLC (3:4:3 ethyl acetate:isopropyl alcohol:water) shows the clean formation of a major product. The precipitated solids are filtered off, washed with anhydrous methanol (4 X 1ml) and the combined filtrates are evaporated under reduced pressure @ 40 °C to give N,N'-diacetyl-L-cystine disodium salt (2) as white solid (688mg, 90% yield, >95% purity). TLC and 1H NMR of this product is identical to a N,N'-diacetyl-L-cystine disodium salt reference standard.

In an alternative, the isolated N,N'-diacetyl-L-cystine disodium salt product from the reaction mixture contains cystine disodium salt, N-acetyl-L-cystine sodium salt or sodium acetate, or a mixture thereof.

### DETAILED DESCRIPTION OF THE INVENTION

Except in the examples, or where otherwise explicitly indicated, all numbers in this description indicating amounts of material or conditions of reaction, physical properties of materials and/or use are to be understood as modified by the word "about."

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

The present invention obviates the needs of the prior art by providing a chemical process to specifically prepare N,N'-diacetyl-L-cystine (NDAC) disodium salt (2) directly from L-cystine (1) using an acetylating agent (e.g. acetyl chloride or acetic anhydride).

The chemical process of the present invention is quick and is performed in a single vessel in one step, when a batch process is used. With either batch or continuous process, the inventive reaction advantageously starts with cystine, and uses alcohol-based solvents which can be recycled. There are no undesirable cystine-derived by-products generated and any by-products generated consists of innocuous salts (e.g. NaCl) that can be conveniently filtered off and recycled or small amounts of acceptable by-products consisting of cystine disodium salt (from unreacted cystine), N-acetyl-L-cystine sodium salt (resulting from monoacetylation of cystine) and sodium acetate. The N,N'-diacetyl-L-cystine (NDAC) disodium salt (2) product is isolated upon solvent evaporation, with no purification needed.

### N,N'-diacetyl-L-cystine disodium salt ("NDAC disodium salt")

Applicants have discovered that N,N'-diacetyl-L-Cystine ("NDAC") and NDAC disodium salts are useful amides in personal care compositions. In skin cosmetic compositions, NDAC and its disodium salts, when applied to skin, are converted to cysteine intracellularly. Cysteine in turn has a number of cosmetic uses, including as a glutathione precursor. Applicants have discovered that NDAC disodium salt has superior functional benefits for ultimate delivery of cysteine for cosmetic uses.

NDAC salt is a disodium salt of N,N-diacetyl cystine (Also referred to as N,N'-diacetyl-L-cystine or NDAC). NDAC structure is shown below (C₁₀H₁₆N₂O₆S₂):

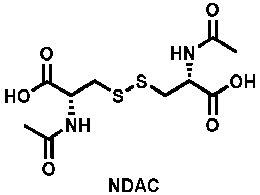

The molecular weight of NDAC = 324.4.

N,N'-diacetyl-L-Cystine ("NDAC") disodium salt of Formula (2), prepared according to the inventive process, has the following general structure (C₁₀H₁₄N₂Na₂O₆S₂):

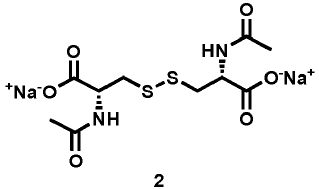

The molecular weight of NDAC disodium salt = 368.3.

NDAC or its disodium salt is not readily commercially available in large scale. N,N'-diacetyl-L-cystine disodium salt ("NDAC disodium salt") may be prepared according to the inventive process. The inventive process is most useful for the synthesis of NDAC disodium salt by using L-cystine (1), which is readily available commercially in bulk quantities.

### Inventive Process

According to the inventive process, N,N'-diacetyl-L-Cystine **salts** (NDAC salts) are prepared in a single step.

The process starts with dispensing a solution of **hydroxyalkane (about 0.5ml to about 100ml hydroxyalkane per mmol of L-cystine),** such as for example methanol, ethanol or isopropanol (preferably methanol, MeOH) or a dihydroxyalkane such as for example propylene glycol, or a trihydroxyalkane such as for example glycerol.

The process includes adding an **alkali metal hydroxide,** or **alkaline earth hydroxides or oxides** (4-6 molar equivalents relative to L-cystine) to the solution, thereby producing a mixture of hydroxyalkane and alkali metal hydroxide and/or alkaline earth hydroxides or oxides in solution. Alkaline earth hydroxides or oxides include for example group II alkali earth metals such as (Mg, Ca and Ba), preferably calcium hydroxide or calcium oxide. Other bases that could be used are the hydroxides, carbonates, bicarbonates and oxides of selected group I alkali metals (such as Li, K and Cs). Alkali metal hydroxide preferably include halide metal hydroxide, most preferably for example sodium hydroxide or potassium hydroxide. Particularly preferred are sodium hydroxide, sodium carbonate and sodium bicarbonate, because they generate non-toxic sodium chloride as the only by-product in solution which is easily separated from the product via filtration. Sodium hydroxide is most preferred because it is the strongest base of the three, leading to more efficient reaction rates.

The process includes adding Cystine (1 molar equivalent, i.e., 500mg, 2.1mmol) to the solution mixture.

The process includes maintaining the solution at temperatures ranging between 3-50 °C (preferably between 5-35 °C, more preferably between 5-25 °C, even more preferably less than about 10 °C, most preferably at about 5 °C. At this point, the process includes stirring the solution mixture between 5 min to 2h.

Subsequently, the process includes cooling the solution to between about 5-25 °C, depending on the initial temperature referenced above.

Next, the process includes adding an acetylating agent to the cooled solution mixture slowly or in portions. An acetylating agent includes for example acetyl chloride or acetic anhydride (preferably acetyl chloride, AcCI) (2-4 molar equivalents with respect to L-cystine).

The process includes maintaining the temperature below 50 °C (preferably below 30 °C, more preferably below 20 °C, most preferably less than about 10 °C ), depending on the type of acetylating agent used to achieve the chemical transformation on the nitrogen groups of L-cystine. In one embodiment, acetyl chloride (AcCI) is added at room temperature (R.T.) of about 15 °C to about 20°C.

The process includes stirring the reaction mixture until all the cystine is consumed (typically between 15min-24h) and the desired N,N'-diacetyl-L-cystine disodium salt is generated. Upon reacting cystine with acetyl chloride, in the presence of sodium hydroxide in methanol, N,N'-diacetyl-L-cystine disodium salt, i.e., compound of formula (2) is obtained as a solution in methanol and the innocuous by-product sodium chloride ("NaCl") precipitates out of the reaction mixture. Any small amounts of unreacted cystine, monoacetylated cystine (N-acetyl-L-cystine) or sodium acetate impurities will remain in the methanol solution.

Upon completion of the reaction, the process includes filtering off the precipitated NaCl solids and washing them with a hydroxyalkane (preferably an alcohol, most preferably methanol), followed by allowing the combined filtrates to evaporate, to yield N,N'-diacetyl-L-cystine disodium salt as a solid. Small amounts of impurities that may be present in the N,N'-diacetyl-L-cystine disodium salt include cystine disodium salt, N-acetyl-L-cystine sodium salt and sodium acetate.

All reagents suitable for inventive process are available from commercial sources.

The relative amounts of cystine and a base are such as to not have excessive starting ingredients or by-product salts upon reaction completion. According to the inventive process, about 0.9 to 1 molar equivalent of cystine to 4 to 6 molar equivalents of a base are preferred, preferably 1.0 molar equivalents of cystine to 4 molar equivalents of base.

Sufficient hydroxyalkane and base are used in the inventive process to generate a basic solution [as determined by measuring the pH of a solution prepared using an aliquot of said hydroxyalkane/base solution (2 parts) with water (8 parts)] with an apparent pH of between 9-15. Enough equivalents of base are added to 1) deprotonate the amine group of cystine, thereby making it nucleophilic enough, to allow it to react with acetyl chloride and 2) neutralize the HCl generated from the reaction of amine with acetyl chloride. The stronger the base, the higher the pH and the faster the reaction time.

In the penultimate step of the process, the precipitated by-product salts (e.g. "NaCl") are separated and isolated from the filtrate containing the NDAC disodium salt product, by centrifugation or filtering, preferably by filtering. The isolated by-product salts are optionally washed with additional hydroxyalkane and all filtrates containing the NDAC disodium salt product are combined.

In the last step of the process, the combined filtrates are evaporated, leaving the desired NDAC disodium salt product as a solid in pure form. Preferably the inventive process also comprises a further step, wherein the evaporated alcohol/methanol solvent from the combined filtrates is condensed and reused and the isolated by-product salts are recycled

The inventive process is advantageous, at least because it uses greener solvents relative to other solvents used in the industry to carry out amidations using these specific types of reactants, results in no undesirable cystine-derived by-products and only innocuous by-products salts (NaCl) that can be recycled or acceptable by-products (cystine disodium salt, N-acetyl-L-cystine sodium salt and sodium acetate), the solvents can be fully reused, and is relatively fast. It also results in improved purity of from 90% to 99%, preferably from 95% to 99%, and most preferably at least 98% to 99%, and improved yield from 85% to 99%, preferably from 90% to 99%, and most preferably at least 95% to 99%.

### Examples

### Experimental Methods

All reagents and solvents were obtained from commercial sources and used without further purification. L-Cystine (>98%), acetyl chloride (98%), sodium hydroxide (97%) and anhydrous methanol (99.8%) were purchased from Sigma. N,N'-diacetyl-L-cystine (NDAC; 95%) was purchased from CombiBlocks..

### Reaction Monitoring Methods

Reaction monitoring was performed using thin layer chromatography (TLC) on silica gel using mixtures of ethyl acetate, isopropyl alcohol and water. Visualization of TLC plates was performed by subjecting TLC plates to 2% ninhydrin in ethanol followed by heat. Qualitative analysis and confirmation of reaction products was performed using 1H nuclear magnetic resonance (1H NMR) and liquid chromatography mass spectrometry (LCMS). Purity of reaction products was assessed via comparison of pure authentic standards using a combination of TLC, 1H NMR and LCMS. Product identity was confirmed via comparison with an authentic NDAC disodium salt reference standard using 1H NMR, TLC and LCMS.. A reference standard of NDAC disodium salt was prepared by dissolving NDAC (100mg, 0.3mmol) in water (1ml), adding sodium bicarbonate (51mg, 0.6mmol) and stirring for 10min. The solvent was removed under reduced pressure at 50 °C to give pure NDAC disodium salt as a white powder (111mg, 98%, >95% purity).

### EXAMPLE 1

An example of the process within the scope of the invention was performed. The chemical reaction scheme is shown below.

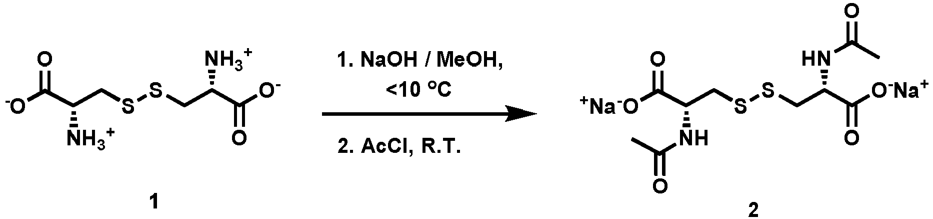

A specific experimental procedure was carried out in one step to prepare N,N'-diacetyl-L-cystine disodium salt, as follows.

Cystine (1) (500mg, 2.1mmol) was added to a cold (~10 °C) solution of methanol (5ml) containing sodium hydroxide (333mg, 8.3mmol) and stirred for 5 min.

The solution was cooled to 5 °C and acetyl chloride (0.3ml, 4.2mmol) added portionwise, maintaining the temperature below 10 °C.

The resulting white suspension was stirred at 5 °C for 20 min and allowed to warm up to R.T. (~20 °C) over 10 min.

At this time, TLC (3:4:3 ethyl acetate: isopropyl alcohol:water) showed the clean formation of a major product.

The precipitated solids were filtered off, washed with anhydrous methanol (4 X 1ml) and the combined filtrates evaporated under reduced pressure @ 40 °C to give N,N'-diacetyl-L-cystine disodium salt (2) as white solid (688mg, 90% yield).

Product identity and purity (>95%) was confirmed by 1H NMR, TLC and LCMS via comparison with an authentic NDAC disodium salt reference standard.

## Claims

1. A process of making N,N'-diacetyl-L-Cystine disodium salt, the process comprising:
(i) Mixing hydroxyalkane (between 0.5-100Lper mol of cystine) with a sodium base (4.0 molar equivalents per mole of cystine) to form a cold solution at a temperature of from 5 to 10°C;
(ii) Adding cystine (1 molar equivalent) to said cold solution and stirring, for a sufficient time, to form a basic cystine solution;
(iii) Optionally, Cooling the cystine solution to 5°C;
(iv) Adding acetyl chloride (2 molar equivalents per mole of cystine) portionwise, while maintaining the temperature between 3 and 50 °C, thereby resulting in a white suspension;
(v) Stirring said white suspension and allowing said suspension to warm up to a room temperature of 15°C to 50°C,
thereby resulting in N,N'-diacetyl-L-Cystine disodium salt product dissolved in solution and sodium chloride by-product precipitated solid in said suspension.

2. The process according to claim 1, further comprising separating said N,N'-diacetyl-L-Cystine disodium salt product from the reaction mixture.

3. The process according to claim 1, wherein the process further comprises:
Filtering off said sodium chloride by-product precipitated solid, thereby separating said precipitated solid away from N,N'-diacetyl-L-Cystine disodium salt product dissolved in filtrate,
Washing said precipitated solid with anhydrous methanol and collecting said methanol filtrate,
Combining all filtrates,
Evaporating said combined filtrate under reduced pressure and temperature of 40 °C, resulting in N,N'-diacetyl-L-Cystine disodium salt product as white solid at 90% yield.

4. The process according to any of the preceding claims, wherein the **hydroxyalkane** is methanol, ethanol or isopropanol, preferably methanol.

5. The process according to any of the preceding claims, wherein the base selected from the group consisting of alkali metal hydroxides, carbonates, and mixtures thereof; preferably sodium hydroxide.

6. The process according to any one of the preceding claims, wherein said **stirring cystine in solution** for said sufficient time takes 5 minutes to 1 hour, preferably 5 minutes.

7. The process according to any one of the preceding claims, wherein **stirring said white suspension** takes 10 minutes to 24 hours, preferably 15 minutes to 24 hours, more preferably 20 minutes.

8. The process according to any one of the preceding claims, wherein **warming** said white suspension to Room Temperature takes 10 min to 30 min, preferably 10 min.

9. The process according to any one of claims 1 to 7 wherein total reaction completion takes from 15 minutes to 25 hours, preferably 35 minutes.

10. A chemical process of making N-N'-di-acetyl-cystine disodium salt ("NDAC salt"), the process comprising the steps:
Forming a reaction mixture, starting with cystine in alcohol solvent in the presence of sodium hydroxide;
Acetylating said cystine with an acetylating agent; and
Isolating said N,N'-diacetyl-L-Cystine disodium salt product from said reaction mixture.

11. The process according to claim 10, further comprising recycling said alcohol based solvent.

12. The process according to claim 10, wherein said solvent is methanol.

13. The process according to claim 10, wherein said acetylating agent is acetyl chloride or acetic anhydride.e.

14. The process according to claim 10, wherein said acetylating agent is used in an amount of 2 to 4 molar equivalents with respect to said cystine..

15. The process according to any of the claims 10 to 14, wherein said isolated N,N'-diacetyl-L-cystine disodium salt product from said reaction mixture contains cystine disodium salt, N-acetyl-L-cystine sodium salt or sodium acetate, or a mixture thereof.

## Patentansprüche

1. Verfahren zur Herstellung von N,N'-Diacetyl-L-Cystin-Dinatriumsalz, wobei das Verfahren umfasst:
(i) Mischen von Hydroxyalkan (zwischen 0,5-100 I pro Mol Cystin) mit einer Natriumbase (4,0 Moläquivalente pro Mol Cystin), um eine kalte Lösung bei einer Temperatur von 5 bis 10°C zu bilden;
(ii) Zugeben von Cystin (1 Moläquivalent) zu der kalten Lösung und ausreichend langes Rühren, um eine basische Cystinlösung zu bilden;
(iii) optional Kühlen der Cystinlösung auf 5°C;
(iv) portionsweises Zugeben von Acetylchlorid (2 Moläquivalente pro Mol Cystin), während die Temperatur zwischen 3 und 50°C gehalten wird, was zu einer weißen Suspension führt;
(v) Rühren der weißen Suspension und Aufwärmenlassen der Suspension auf Raumtemperatur von 15° bis 50°C,
wodurch ein N,N'-Diacetyl-L-Cystin-Dinatriumsalzprodukt, gelöst in der Lösung, und ein Natriumchlorid-Nebenprodukt in der Suspension, ausgefällt als Feststoff, entstehen.

2. Verfahren nach Anspruch 1, ferner umfassend das Abtrennen des N,N'-Diacetyl-L-Cystin-Dinatriumsalzprodukts aus der Reaktionsmischung.

3. Verfahren nach Anspruch 1, wobei das Verfahren ferner umfasst:
Abfiltrieren des als Feststoff ausgefällten Natriumchlorid-Nebenprodukts,
wodurch der ausgefällte Feststoff von dem im Filtrat gelösten N,N'-Diacetyl-L-Cystin-Dinatriumsalzprodukt abgetrennt wird,
Waschen des ausgefällten Feststoffs mit wasserfreiem Methanol und Sammeln des Methanolfiltrats,
Kombinieren sämtlicher Filtrate,
Eindampfen des kombinierten Filtrats unter vermindertem Druck und bei einer Temperatur von 40°C, was zu einem N,N'-Diacetyl-L-Cystin-Dinatrium-salzprodukt als weißer Feststoff in einer Ausbeute von 90% führt.

4. Verfahren nach einem der vorhergehenden Ansprüche, wobei das Hydroxyalkan Methanol, Ethanol oder Isopropanol, vorzugsweise Methanol, ist.

5. Verfahren nach einem der vorhergehenden Ansprüche, wobei die Base aus der aus Alkalimetallhydroxiden, Carbonaten und Mischungen davon, vorzugsweise Natriumhydroxid, bestehenden Gruppe ausgewählt ist.

6. Verfahren nach irgendeinem der vorhergehenden Ansprüche, wobei das Rühren von Cystin in Lösung für eine ausreichend lange Zeit 5 Minuten bis zu 1 Stunde, vorzugsweise 5 Minuten, dauert.

7. Verfahren nach irgendeinem der vorhergehenden Ansprüche, wobei das Rühren der weißen Suspension 10 Minuten bis 24 Stunden, vorzugsweise 15 Minuten bis 24 Stunden, besonders bevorzugt 20 Minuten, dauert.

8. Verfahren nach einem der vorhergehenden Ansprüche, wobei das Erwärmen der weißen Suspension auf Raumtemperatur 10 min bis 30 min, vorzugsweise 10 min, dauert.

9. Verfahren nach einem der Ansprüche 1 bis 7, wobei der vollständige Reaktionsabschluss 15 Minuten bis 25 Stunden, vorzugsweise 35 Minuten, dauert.

10. Chemisches Verfahren zur Herstellung von N,N'-Diacetyl-Cystin-Dinatriumsalz ("NDAC-Salz"), wobei das Verfahren die Schritte umfasst:
Bilden einer Reaktionsmischung, beginnend mit Cystin in alkoholischer Lösung in Gegenwart von Natriumhydroxid;
Acetylieren des Cystins mit einem Acetylierungsmittel; und
Isolieren des N,N'-Diacetyl-L-Cystin-Dinatriumsalzprodukts aus der Reaktionsmischung.

11. Verfahren nach Anspruch 10, ferner umfassend die Rückgewinnung des Lösungsmittels auf Alkoholbasis.

12. Verfahren nach Anspruch 10, wobei das Lösungsmittel Methanol ist.

13. Verfahren nach Anspruch 10, wobei das Acetylierungsmittel Acetylchlorid oder Essigsäureanhydrid ist.

14. Verfahren nach Anspruch 10, wobei das Acetylierungsmittel in einer Menge von 2 bis 4 Moläquivalenten, bezogen auf das Cystin, verwendet wird.

15. Verfahren nach einem der Ansprüche 10 bis 14, wobei das aus der Reaktionsmischung isolierte N,N'-Diacetyl-L-Cystin-Dinatriumsalzprodukt Cystin-Dinatriumsalz, N-Acetyl-L-Cystin-Natriumsalz oder Natriumacetat oder eine Mischung davon enthält.

## Revendications

1. Procédé de production du sel disodique de N,N'-diacétyl-L-cystine, le procédé comprenant :
(i) Le mélange d'hydroxyalcane (entre 0,5 et 100 l par mole de cystine) avec une base de sodium (4,0 équivalents molaires par mole de cystine) pour former une solution froide à une température de 5 à 10 °C ;
(ii) L'ajout de cystine (1 équivalent molaire) à ladite solution froide et l'agitation, pendant une durée suffisante, pour former une solution de cystine basique ;
(iii) Facultativement, le refroidissement de la solution de cystine à 5 °C ;
(iv) L'ajout de chlorure d'acétyle (2 équivalents molaires par mole de cystine) par portions, tout en maintenant la température entre 3 et 50 °C, résultant ainsi en une suspension blanche ;
(v) L'agitation de ladite suspension blanche et le fait de permettre à ladite suspension de se réchauffer jusqu'à une température ambiante de 15 °C à 50 °C,
résultant ainsi en un produit de sel disodique de N,N'-diacétyl-L-cystine dissous dans une solution et en un sous-produit de chlorure de sodium solide précipité dans ladite suspension.

2. Procédé selon la revendication 1, comprenant en outre la séparation dudit produit de sel disodique de N,N'-diacétyl-L-cystine du mélange réactionnel.

3. Procédé selon la revendication 1, dans lequel le procédé comprend en outre :
L'extraction par filtration dudit sous-produit de chlorure de sodium solide précipité, séparant ainsi ledit solide précipité du produit de sel disodique de N,N'-diacétyl-L-cystine dissous dans un filtrat,
Le lavage dudit solide précipité avec du méthanol anhydre et la collecte dudit filtrat de méthanol,
La combinaison de tous les filtrats,
L'évaporation dudit filtrat combiné sous pression réduite et à une température de 40 °C, résultant en un produit de sel disodique de N,N'-diacétyl-L-cystine en tant que solide blanc à un rendement de 90 %.

4. Procédé selon l'une quelconque des revendications précédentes, dans lequel l**'hydroxyalcane** est du méthanol, de l'éthanol ou de l'isopropanol, de préférence du méthanol.

5. Procédé selon l'une quelconque des revendications précédentes, dans lequel la **base** est choisie dans le groupe constitué d'hydroxydes de métal alcalin, de carbonates, et de mélanges de ceux-ci ; de préférence d'hydroxyde de sodium.

6. Procédé selon l'une quelconque des revendications précédentes, dans lequel ladite **agitation de cystine en solution** pendant une durée suffisante prend 5 minutes à 1 heure, de préférence 5 minutes.

7. Procédé selon l'une quelconque des revendications précédentes, dans lequel l**'agitation de ladite suspension blanche** prend 10 minutes à 24 heures, de préférence 15 minutes à 24 heures, de manière davantage préférée 20 minutes.

8. Procédé selon l'une quelconque des revendications précédentes, dans lequel le **réchauffement** de ladite suspension blanche jusqu'à la température ambiante prend 10 min à 30 min, de préférence 10 min.

9. Procédé selon l'une quelconque des revendications 1 à 7 dans lequel la réalisation de la réaction totale prend de 15 minutes à 25 heures, de préférence 35 minutes.

10. Procédé chimique de production du sel disodique de N-N'-diacétyl-L-cystine (« sel NDAC »), le procédé comprenant les étapes de :
Formation d'un mélange réactionnel, en commençant par de la cystine dans un solvant d'alcool en présence d'hydroxyde de sodium ;
Acétylation de ladite cystine avec un agent d'acétylation ; et
Isolement dudit produit de sel disodique de N,N'-diacétyl-L-cystine dudit mélange réactionnel.

11. Procédé selon la revendication 10, comprenant en outre le recyclage dudit solvant à base d'alcool.

12. Procédé selon la revendication 10, dans lequel ledit solvant est du méthanol.

13. Procédé selon la revendication 10, dans lequel ledit agent d'acétylation est du chlorure d'acétyle ou de l'anhydride acétique.

14. Procédé selon la revendication 10, dans lequel ledit agent d'acétylation est utilisé en une quantité de 2 à 4 équivalents molaires par rapport à ladite cystine.

15. Procédé selon l'une quelconque des revendications 10 à 14, dans lequel ledit produit de sel disodique de N,N'-diacétyl-L-cystine isolé dudit mélange réactionnel contient un sel disodique de cystine, un sel sodique de N-acétyl-L-cystine ou de l'acétate de sodium, ou un mélange de ceux-ci.
